# EUROPEAN PATENT APPLICATION

(11) **EP 1 870 111 A1**
(43) Date of publication of application: **26.12.2007**
(21) Application number: 06115775.6
(22) Date of filing: 21.06.2006
(51) Int. Cl.: A61L 2/18, A61L 2/24, A61L 2/26, F16K 21/00, F16K 1/38

(54) **Inlet device for disinfection apparatus and method for liquid transfer**

(71) Applicant: Getinge Disinfection AB, 351 15 Växjö (SE)
(72) Inventor: Johansson, Jonas, SE-352 45, Växjö (SE)
(74) Representative: Kraenzmer, Martin

(57) **Abstract**

The present invention relates to a liquid disinfection apparatus for cleaning of objects, such as health care objects, which disinfection apparatus comprises; a chamber (3) which is arranged to receive said objects for cleaning, a stand-by tank (5) adapted to receive liquid to be used in said chamber for cleaning, a spray system (17) comprising nozzles for distributing liquid in said chamber,
wherein said stand-by tank (5) is liquid flow connected with said chamber (3) via a rapid transfer port (6) so as to constitute an immediate supply of a predetermined liquid volume from the stand-by tank (5) to said chamber (3) without passing said nozzles, wherein the spray system (17) is arranged to subsequently distribute said predetermined volume, being received in the chamber (3), for the cleaning of said objects. The present invention also relates to a method for liquid transfer at said disinfection apparatus.

## Description

### Field of the Invention

The present invention relates to a liquid disinfection apparatus for cleaning of objects, such as health care objects, which disinfection apparatus comprises a chamber which is arranged to receive the objects for cleaning, a stand-by tank adapted to receive liquid to be used in the chamber for cleaning, and a spray system comprising nozzles for distributing liquid in the chamber. The invention also relates to a method for liquid transfer of a disinfection apparatus.

### Background Art

Disinfection apparatus of the above type are well known and are also called washer disinfectors. They are are regulated under disinfection standards. Washer disinfectors are used for cleaning and disinfection of goods, instruments and other objects that are used in, for instance, hospitals, laboratories and in the pharmaceutical industry. As examples of such objects, mention can be made of vessels of different kinds, instruments containers, surgery equipments, instruments, machine parts in nursing applications and other related objects.

The liquid used in a disinfector may be supplied in different known ways. The major part of liquid used in a disinfector is normally water. One known way is to supply the water directly from the public water system through a water supply line, regulated by a filling valve, such as a magnetic valve, into the chamber of the disinfector. However the pressure, the flow and other parameters may vary between different locations and countries, wherein such parameters may be troublesome to control, especially if a short process time is desired.

Another known way is that the water and possibly other liquids are supplied to a water tank, whereby a supply pump pumps the liquid to spray arms in the chamber of the disinfector. The liquid is collected in the bottom of the chamber, normally in a sump provided with a sump pump for recirculation to the water tank. However, the pumping operations are troublesome, for instance due to the risk of cavitation, especially if a reduced liquid volume is desired. Another issue is that such a disinfector process with satisfactory disinfection result is time-consuming.

Also, different kind of pumps have been tested in disinfectors, such as high pressure pumps. However, such a pumping system may be costly due to the requirement of the system components to withstand such a high pressure.

It is desirable to reduce the process time of a disinfector apparatus. Also, it is suitable to reduce the liquid volume needed in the process. Moreover it is desirable to be able to reduce the number of machine components of the disinfection apparatus and yet maintain its desired disinfection function.

Finally it is advantageous to provide a robust, cost effective and reliable high quality disinfection apparatus.

### Summary of the Invention

The object of the present invention is to provide a disinfection apparatus which allows improvements in relation to prior-art disinfection apparatus in one or more of the above aspects.

In one aspect of the present invention, a disinfection apparatus according to the introduction is further characterised in that the stand-by tank is liquid flow connected with the chamber via a rapid transfer port so as to constitute an immediate supply of a predetermined liquid volume from the stand-by tank to the chamber without passing the nozzles, wherein the spray system is arranged to subsequently distribute the predetermined volume, being received in the chamber, for the cleaning of the objects.

Such a system provides a number of advantages, such as a reduced process time for a disinfection apparatus compared with prior-art technique. Also, it is possible to reduce the number of machine components which may be costly and complex, such as complex pumps. Moreover the noise levels, relating to liquid transfer, may be reduced. Additionally the predetermined liquid volume may be distributed essentially immediately by the spray system after the volume being transferred to the chamber.

The term stand-by tank may be referred to as a tank which is arranged to prepare the liquid to be used and is ready to be used in a specific cleaning phase.

By the expression "rapid transfer port" is meant; a liquid port arranged to immediate transfer liquid from the stand-by tank to the chamber.

By the expression "an immediate supply" is primary meant a substantially direct transfer of liquid and may be an instant and spontaneous transfer. Thus the stand-by tank is arranged in such a way in connection with the rapid transfer port that the liquid is ready to be released to the chamber without using the spray nozzles in the chamber. Thus, the liquid may be transferred without any further flow control. Additionally, the liquid may be transferred from the stand-by tank to the chamber without using any pump.

The outlet of the stand-by tank is preferably arranged to substantially constitute an inlet of the chamber. In use a liquid column may be at least indirectly defined in the stand-by tank to perform a pressure on the transfer port thereby reducing efforts and costs in relation to prior-art technique.

The predetermined volume in the stand-by tank can substantially corresponds to the required liquid volume of a cleaning phase in the chamber. Thus, not only the liquid in itself is prepared, but also a sufficient volume, preferably the water volume for a cleaning phase may be prepared. The cleaning phase may be one of the phases known for a disinfection apparatus such as one of; pre-wash, wash phase, rinse phase and/or disinfection phase. The disinfection phase may be achieved by thermal disinfection and/or by an optional chemical phase with a chemical agent.

The predetermined volume to be transferred may be substantially the whole liquid volume in the stand-by tank. Consequently this enhances and simplifies the regulation of opening and closing of the transfer port. For instance a liquid level indicator may be arranged in the stand-by tank for providing a signal to close the transfer port. An additional advantage is that the volume may be pre-measured.

The chamber is preferably provided with an outlet port to empty the chamber, the port being of a rapid transfer port type. The outlet port preferably having the corresponding advantages, aspects and features as mentioned for the rapid transfer port arranged at the stand-by tank. Thus the process time may be reduced, especially the time relating for supplying and discharging liquids.

The rapid transfer port may be adapted to transfer liquid at a flow velocity; preferably above 1 litres per second, more preferred above 2 litres per second and in particular above 3 litres per second. The values of the flow velocity is regarded as average values, measured from full tank to empty stand-by tank.

The predetermined volume to be transferred may be a volume between 10 and 150 litres, more preferred a volume between 15 to 70 litres and in particular a volume between 20 to 55 litres. Thus, the predetermined volume may be adapted to a so-called walk-in cabinet disinfector, but preferably not to a household dishwasher.

Advantageously, the stand-by tank outlet may be arranged on a higher vertical level than the chamber inlet, wherein the transfer is achieved by means of gravity. Such arrangement may reduce the number of machine components and reduce costs but also ensure for a reliable liquid transfer. The stand-by tank outlet and the chamber inlet may at least partly separate the liquid by a sealing element in a closed position.

The stand-by tank may be liquid flow connected to a separate preparation tank to assist the preparation of the predetermined volume for at least one cleaning phase, e.g. a disinfection phase. Different preparation measures may be performed to reduce the process time, such as one of preheating, premixing and measuring. The preferred liquid is water but alternatively also gases or solid substances may be used during preparation in addition to the liquid.

The spray system may be adapted to distribute air in the chamber in order to dry the objects. Thus a uniform drying may be achieved. Also, at least the elements inside the chamber of the spray system, such as spray arms, spray pipes and/or spray nozzles may be liquid drained.

The stand-by tank may be provided with pressure means to pressurize the liquid to assist liquid transfer to the chamber, when the rapid transfer port is changed from a closed condition to an open condition. The liquid may be pressurized to enhance the transfer for instance by a two part chamber of deviating medium, such as gas and liquid, by an expansion vessel and/or by a pump.

The above objects are also achieved by a method, comprising the steps of;
introducing a first predetermined liquid volume into a stand-by tank,
releasing the first predetermined liquid volume by opening a rapid transfer port to immediately transferring the predetermined liquid volume to a chamber for cleaning of objects,
pumping the liquid from the chamber to a spray system to distribute the first predetermined liquid volume at the objects to be cleaned.

The method achieves corresponding advantages as the disinfection apparatus with reference to the features above. Additionally, the liquid may be pressurized from the pump arranged downstream the chamber to the nozzles in the chamber.

In order to reduce the process time and to prepare for a subsequent phase, the method preferably comprises the step of introducing a second predetermined liquid volume to a stand-by tank when the first predetermined liquid volume is transferred to the chamber.

The method may optionally comprise the step of emptying the first predetermined liquid volume from the chamber by opening an outlet port, the port being of a rapid transfer port type.

### Brief Description of the Drawings

The invention will in the following be described with reference to the accompanying drawings, which for the purpose of exemplification illustrate preferred embodiments of the invention.
Fig. 1 is a schematic sectional view of a disinfection apparatus according to a first embodiment of the invention, seen obliquely from the rear. Fig. 1 also shows a section along I-I of the disinfection apparatus, seen from the side.
Fig. 2 shows in more detail parts of the disinfection apparatus in fig. 1, partly in section, seen obliquely from the rear.
Fig. 3a-3d shows a sequence of liquid transfer of the disinfection apparatus in Fig. 1.
Fig. 4 shows the disinfection apparatus in Fig. 1 in an emptying phase.
Fig. 5a-5b shows a part of a sequence in a disinfection phase, of the disinfection apparatus in Fig. 1.

### Description of Preferred Embodiments

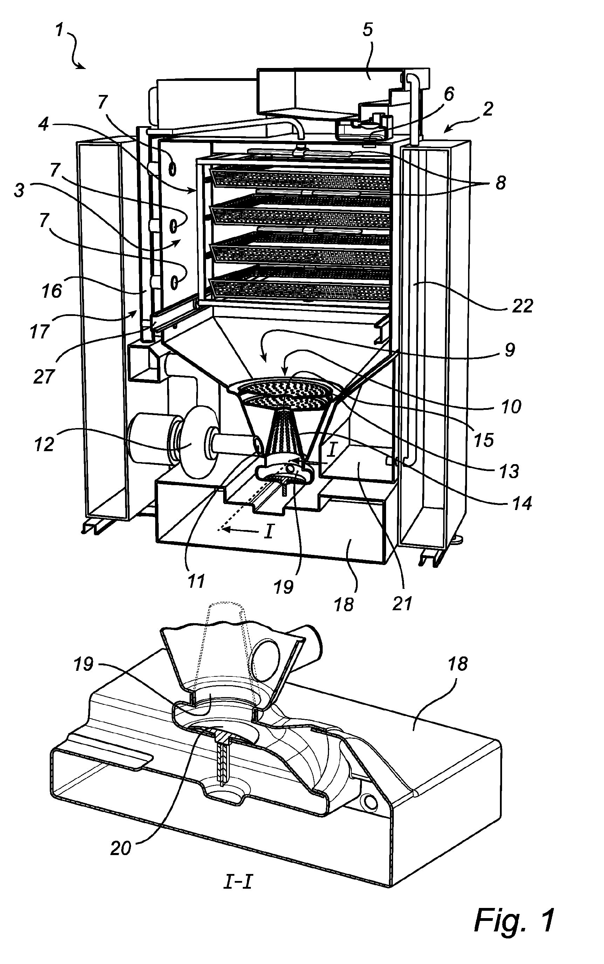

Fig. 1 shows a disinfection apparatus 1 according to a first embodiment of the invention, which has a housing 2 in which a disinfection chamber 3 is arranged. The disinfection apparatus is adapted to perform at least a disinfection phase. The apparatus 1 is preferably an automatic and programmable washer disinfector which may be designed for central sterilization supply departments (CSSD), theatre sterile supply units (TSSU), and sub sterilization units, as well as for laboratories. The following are examples of objects to be washed, disinfected and dried in a disinfection program such as, surgical instruments, endoscopes, instruments for minimum invasive surgery, utensils, anaesthetic and respiratory equipment, as well as bottles, glass jars and hollowware. The chamber 3 is adapted to receive objects to be disinfected which objects may be arranged on a wash cart 4 or on an insert. It is also possible to modify the wash carts 4 or inserts to actual load to almost any type of objects in any specific department.

The disinfector 1 has at least one door (not shown) for sealing the chamber 3 forming an enclosure. The door may be an automated door or for instance a manually operated vertically sliding down door. The apparatus 1 may alternatively be of a pass-through model arranged with two doors.

A stand-by tank 5, in the following called an inlet tank, is provided upstream the chamber to prepare the liquid, which according to this embodiment is essentially water, to be used during the process in the chamber 3. The inlet tank 5 is controllable connected to a water supply pipe (not shown). The chamber 3 is connected to several reservoirs (not shown) for instance for supplying at least one of deionised water, detergents, washing substances/liquids, rinse liquids, deliming agents, disinfection agents or other chemical agents. Such a supply may be achieved by a dosage pump. Thus depending on which program and which phase, for instance in a washing phase, additional agents is supplied to the chamber.

A rapid transfer port 6, in the following called inlet port 6, is arranged in the lower part of the inlet tank 5. The inlet port 6 has a liquid passage which is adapted to transfer a significant volume per time unit, preferably above 2 litres per second.

As can be seen in fig. 1, the wash cart 4 is partly introduced in the chamber 3. The wash cart 4 in the form of a basket rack with multiple levels is guided by rails 27 in the chamber and which wash cart may be liquid/fluid connected in an active position. In this case the chamber exhibits three docking parts 7 for supplying liquid or fluid via corresponding docking parts at the wash cart 4. Spray arms 8, in the form of spray wings, are rotatably arranged in the chamber, in this case arranged at the upper and lower part of the chamber. Spray arms are also provided between the baskets at the wash cart 4. The spray arms have multiple arranged nozzles (not shown). However wash carts or inserts may be of different designs and functions, such as provided with injector heads or ramps adapted for the objects to be received.

Fig. 1 further shows, a filter device 9 which is arranged at a collection space 10, or a sump, in the lower part of the chamber. The collection space 10 is connected to an inlet 11 of a circulation pump 12 for transferring the liquid being received in the chamber to the spray arms 8. The pump 12 pumps liquid from the lower part of the chamber. The filter device 9 has at least one essentially horizontal arranged filter plate 13, in this case two curved filter plates 13 with its concave side facing upwards. A peripheral filter element 14, which may be essentially cylindrical, or as in this case tapered, wherein the element 14 is essentially vertically arranged in the longitudinal direction. The components of the filter device 9 are preferably liquid permeable, and may be made of perforated metal plates or alternatively a filter mesh. The apertures of the filter device 9 is preferably smaller then the apertures of the spray arm nozzles/outlets. For instance the metal perforated plates may have perforations with a 3 mm times 3 mm spacing having an aperture diameter of 2 mm. As seen in fig. 1 the opening of the centre of the plates 13 defines a residue collection space 15 for washed off solids. The residue collection space 15 is preferably provided with a course filter (not shown). The course filter is preferably cup-shaped for collecting larger solid items and detachable for cleaning. The inlet of the circulation pump 12 is arranged transversely and peripherally of the longitudinal direction of the collection space 10. The pump 12 is preferably a so-called low-pressure high-volume pump, in this case a centrifugal pump.

A circulation pipe 16 is arranged downstream the inlet 11 and the circulation pump 12 for circulating the liquid to the spray arms. The circulation pipe 16 is at least partly peripherally arranged with heating elements for heating the liquid which being circulated. The heating may for instance be achieved by steam, by electrical heating elements or by a combination thereof. The liquid flow from the inlet 11 of the circulation pump 12 to the spray arms 8 comprising nozzles defines a spray system 17 for subsequently distributing the liquid in the chamber after that the liquid has been descended into to the chamber 3 from the inlet tank 5. The liquid is preferably pressurized from the pump 12 to the spray arms 8 or to a corresponding device in the chamber 3.

By providing an outlet tank 18 fitted with an outlet port 19, being of a rapid transfer port type, downstream the collection space 10, the process time may be reduced. Alternatively the outlet port 19 may be connected to another device for receiving the liquid used in the chamber, such as a sewer system.

The section I-I in fig 1 shows that the outlet port 19 is in liquid communication with the outlet tank 18. An outlet sealing element 20 is controllably arranged at the outlet port 19 for passage sealing. The outlet sealing element 20 may be a plate valve which is slidingly arranged between an opened and a closed position. In order to save space, the outlet tank 18 may extend more in the horizontal direction than in the vertical direction.

By providing a separate preparation tank 21 connected to the inlet tank 5 (stand-by tank) liquids may be prepared during previous program phases in order to save process time. The separate preparation tank 21 is controllable connected to a water supply conduit (not shown). In this case preparation elements such as heating elements (not shown) are arranged in the preparation tank to prepare the liquid used for the disinfection phase of the disinfection program. As exemplified above other kind of preparations are possible. The liquid, in this case essentially water, is preferably heated to a temperature between 75 to 99°C, more preferably to a temperature between 82 to 97°C and most preferably to a temperature between 85 to 95°C. As the preparation tank 21 is arranged upstream the inlet tank 5, the inlet tank may be disinfected before the liquid is transferred to the chamber 3. In this case the separate preparation tank 21 is arranged at the lower part of the disinfection apparatus. However the separate preparation tank 21 may be arranged at other places, for instance above the chamber and alternatively additionally above the stand-by tank. If a chemical disinfection is performed the temperature may be lower, e.g. approximately 60°C.

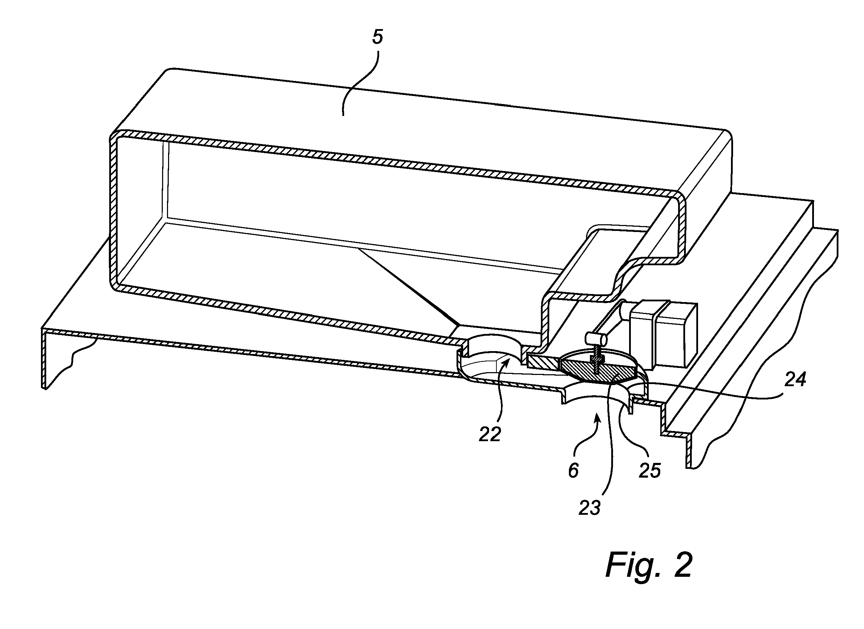

With reference to fig. 2, the interior of the inlet tank 5 has sloping surfaces to a communication section 22. An inlet sealing element 23 is moveably arranged at the inlet port 6 between an opened and a closed position. The sealing element 23 may be arranged with an arm which is pivotable for regulating the liquid transfer through the inlet port 6. However other kind of sealing elements are possible such as a sliding sealing element, a tilting element, a butterfly valve or a piston valve. Preferably, an outlet 24 of the inlet tank 5 constitutes the inlet 25 of the chamber 3, which are separated by the inlet sealing element 23 in the closed position. Thus, as seen in fig 2, the transfer is preferably achieved by means of gravity.

A disinfection program may comprise of different components but usually comprises, at least one of; prewashing, washing, rinsing and/or disinfection. It should be noted that numerous different programs and parameters may be selected by a person skilled in the art.

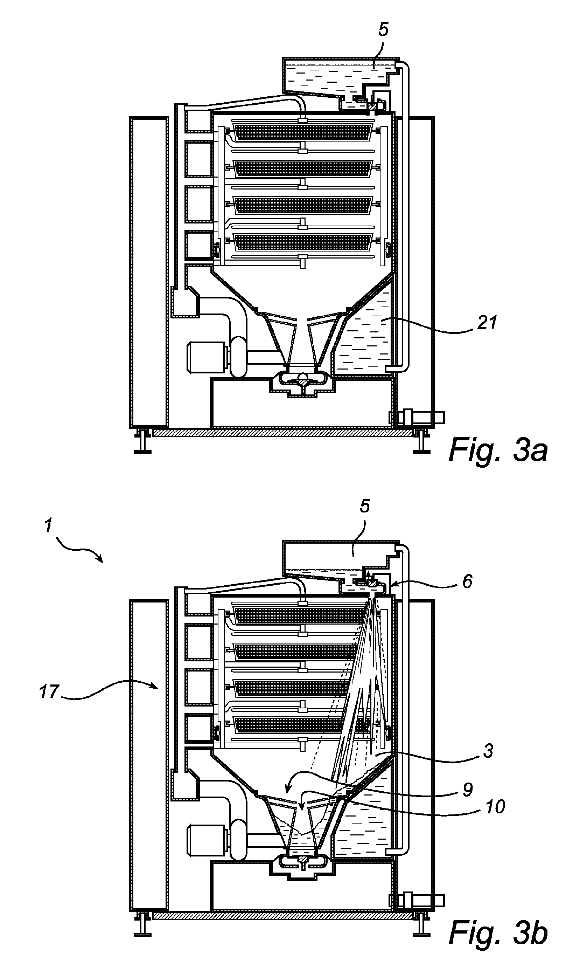

Fig. 3a to fig. 3d shows a sequence of liquid transfer in a general way at the disinfection apparatus as described above. As mentioned above the separate preparation tank 21 is advantageously supplied with, liquid, preferably water, in an early stage for heating the liquid to be used in the disinfection phase.

As seen in fig 3a, the inlet tank 5 is supplied with essentially a predetermined volume, preferably essentially water. The predetermined liquid volume is preferably measured during the supply by a liquid indicator (not shown) arranged in the inlet tank 5.

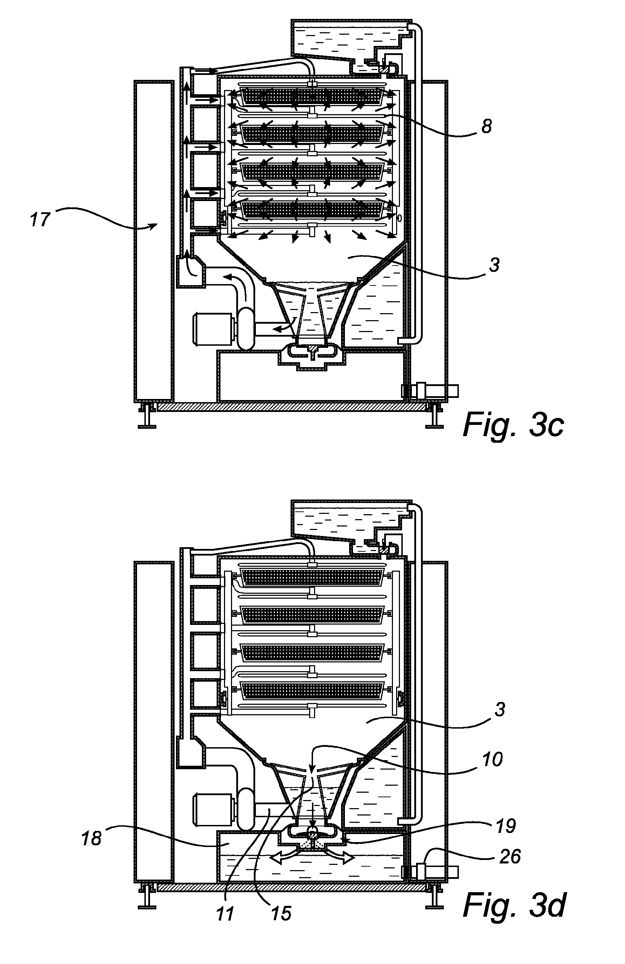

Fig. 3b shows the phases of opening an inlet port 6 for transferring the (first) predetermined liquid volume to the chamber 3. The liquid indicator senses when the inlet port 6 may close. For instance if additional water should be needed for a specific phase, the inlet port 6 and the water supply pipe may remain opened for a selected time. As mentioned above additional cleaning agents may be separately supplied to the chamber depending on the program and the program phase, such as a washing phase. After closing the port 6, to a preferably emty inlet tank 5, a second predetermined liquid volume may be introduced to the stand-by tank 5. However it should be noted that the above introducing phase may be performed during other phases, for instance simultaneously during one of the Fig 3c phases.

Referring to Fig 3c which shows a part of the sequence which preferably is repeated during a selected time which depends on the selected program and the selected parameters. The descended liquid in the chamber is pumped from the chamber 3 to the spray system 17 with spray arms 8. When the liquid being draw into the pump inlet 11, it is filtered by the filter device 9 arranged at the collection space 10 in the chamber.

The liquid may be heated at least along a part of the path between the chamber 3 and the spray arm 8. In this case the liquid is heated at the circulation pipe 16 by heating elements. The liquid is subsequently distributed and spread by the spray arms 8 or by similar nozzle device within the chamber 3.

When the predetermined liquid volume being circulated a selected time, the shown phase in fig 3d is performed;
opening an outlet port 19 for transferring the predetermined liquid volume to the outlet tank 18. The filter device is preferably cleaned to some extent by the flow of the liquid transfer from the chamber 3 to the outlet tank 19. As mentioned above larger items are received in the residue collection space 15 by the course filter. Afterwards the outlet port 19 is closed after a preset time or an indicted absence of liquid in the chamber 3.

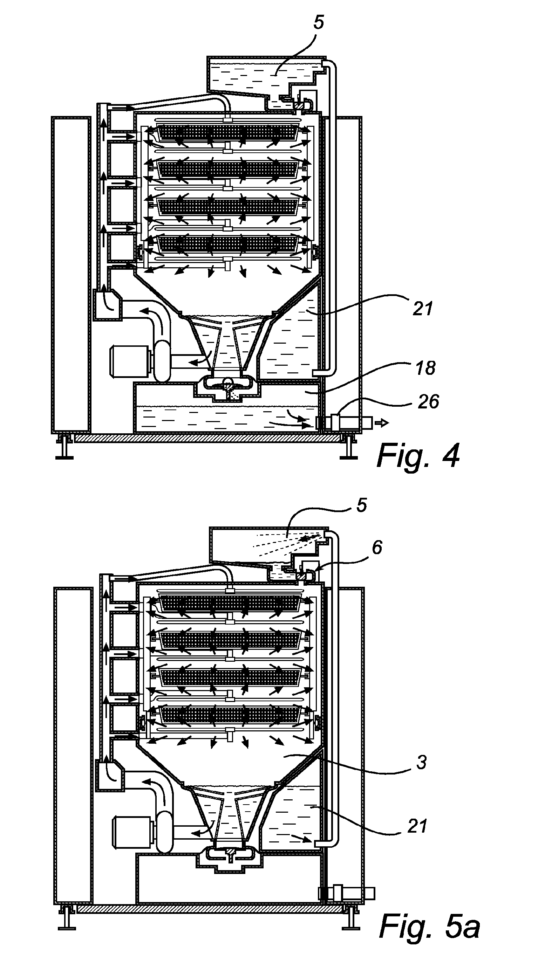

Fig 4 shows an exhaust pump 26 connected to a sewer system (not shown) to exhaust the liquid from the outlet tank 18. Such operation may be performed during a suitable time gap, preferably when no additional liquid is supplied to the outlet tank, for instance during one of the following phases; when liquid is supplied to the chamber or the inlet tank, when liquid is circulated by the circulation pump 12 or after the final phase in a selected program.

Fig 5a-5b shows an example of how a separate preparation tank 21 may be used according to the invention. As the heating of water for a disinfection phase is time consuming, the separate preparation tank is preferably supplied with water in an early stage of the program or alternatively already in the previous program.

The transfer of liquid from the preparation tank 21 to the inlet tank 5 may be performed during an active phase of a program, such as when liquid being circulated in the chamber as in fig 5a. Alternatively, the sequence shown in 5a-5b may be performed during an inactive phase.

The liquid transfer of the disinfection apparatus is carried out by transferring a predetermined liquid volume, from the preparation tank 21 to the inlet tank 5. In this case the transfer is achieved by pumping the liquid.

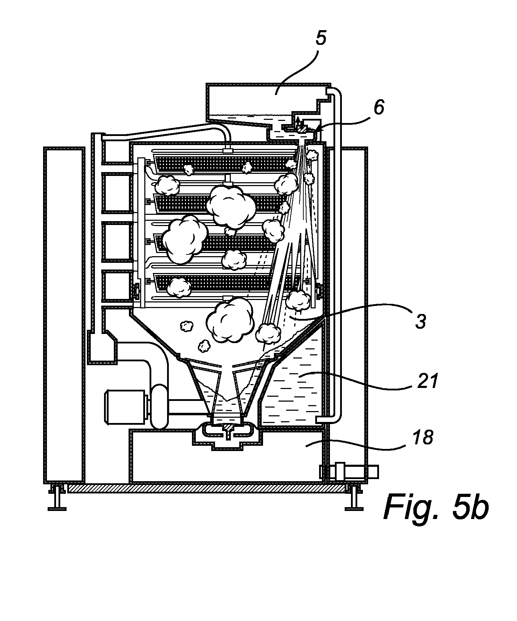

Subsequently, the inlet tank may be disinfected during a preset time. Afterwards the inlet port 6 is opened for transferring the predetermined liquid volume to the chamber 3 (see fig 5b). Subsequently the interior of the chamber 3 and the present objects are disinfected, preferably by hot liquid disinfection as described above.

The liquid transfer in the disinfection phase may be performed essentially in the same way as described above, according to the sequence in fig 3c showing when the liquid is being circulated and in fig. 3d showing the transfer to outlet tank 18. Depending on the selected program or the selected subsequent program or phase, the inlet tank 5 and/or the separate preparation tank 21 may be empty or alternatively at least partly refilled. For instance a predetermined liquid volume may be introduced into the preparation tank 21 to prepare for the next program as shown in fig. 5b.

Partly referring to fig. 4, the liquid transferred to the outlet tank 18 is preferably pumped to the sewer by the sewer pump 26 when the liquid is below a predetermined liquid temperature. Alternatively the temperature may be adjusted by mixing of colder liquids.

Also, the pH-value of the liquid in the outlet tank may be adjusted by pH-regulating liquid/substance, if the pH-value is diverging from an accepted level after one of the cleaning phases, for instance after a so-called chemical phase.

It will be appreciated that the above-described embodiment of the invention can be modified and varied by a person skilled in the art without departing from the inventive concept defined in the claims. For instance, it is possible that the inlet tank 5 is disinfected as a primary phase when the apparatus has been inactive for a selected number of hours.

It is also possible that the outlet of the preparation tank is arranged on a higher vertical level than the stand-by tank inlet, wherein the transfer is achieved by means of gravity.

## Claims

1. A liquid disinfection apparatus for cleaning of objects, such as health care objects, which disinfection apparatus comprises,
a chamber (3) which is arranged to receive said objects for cleaning,
a stand-by tank (5) adapted to receive liquid to be used in said chamber for cleaning,
a spray system (17) comprising nozzles for distributing liquid in said chamber,
**characterised in that** said stand-by tank (5) is liquid flow connected with said chamber (3) via a rapid transfer port (6) so as to constitute an immediate supply of a predetermined liquid volume from the stand-by tank (5) to said chamber (3) without passing said nozzles, wherein the spray system (17) is arranged to subsequently distribute said predetermined volume, being received in the chamber (3), for the cleaning of said objects.

2. The disinfection apparatus as claimed in claim 1, wherein an outlet (24) of said stand-by tank (5) is arranged to constitute an inlet (25) of said chamber (3).

3. The disinfection apparatus as claimed in claim 1 or 2, wherein said predetermined volume in the stand-by tank (5) essentially corresponds to the required liquid volume of a cleaning phase in said chamber (3).

4. The disinfection apparatus as claimed in any one of the preceding claims, wherein said predetermined volume to be transferred is substantially the whole liquid volume in said stand-by tank (5).

5. The disinfection apparatus as claimed in any one of the preceding claims, wherein said chamber (3) is provided with an outlet port (19) to empty the chamber (3), said port being of a rapid transfer port type.

6. The disinfection apparatus as claimed in any one of the preceding claims, wherein said rapid transfer port (6) is adapted to transfer liquid at a flow velocity; preferably above 1 litres per second, more preferably above 2 litres per second and most preferably above 3 litres per second.

7. The disinfection apparatus as claimed in any one of the preceding claims, wherein said predetermined volume to be transferred is a volume between 10 and 150 litres, more preferably a volume between 15 to 70 litres and most preferably a volume between 20 to 55 litres.

8. The disinfection apparatus as claimed in any one of the preceding claims, wherein the stand-by tank outlet (24) is arranged on a higher vertical level than the chamber inlet (25), wherein the transfer is achieved by means of gravity.

9. The disinfection apparatus as claimed in any one of the preceding claims, wherein the stand-by tank (5) is liquid flow connected to a separate preparation tank (21) to assist the preparation of the predetermined volume for at least one cleaning phase, e.g. a disinfection phase.

10. The disinfection apparatus as claimed in any one of the preceding claims, wherein said spray system (17) is adapted to distribute air in said chamber (3) in order to dry said objects.

11. The disinfection apparatus as claimed in any one of the preceding claims, wherein the stand-by tank is provided with pressure means to pressurize the liquid to assist liquid transfer to said chamber (3), when the rapid transfer port (6) is changed from a closed condition to an open condition.

12. A method for liquid transfer of a disinfection apparatus, comprising the steps of; introducing a first predetermined liquid volume to a stand-by tank (5),
releasing said first predetermined liquid volume by opening a rapid transfer port (6) to immediately transferring the predetermined liquid volume to a chamber (3) for cleaning of objects,
pumping the liquid from the chamber (3) to a spray system (17) to distribute said first predetermined liquid volume at the objects to be cleaned.

13. Method as claimed in claim 12, further comprising the step of; introducing a second predetermined liquid volume to a stand-by tank (5) when said first predetermined liquid volume is transferred to said chamber (3).

14. Method as claimed in claim 12 or 13, further comprising the step of;
emptying the first predetermined liquid volume from said chamber (3) by opening an outlet port (19), said port being of a rapid transfer port type.
